(19) Europäisches Patentamt
European Patent Office
Office européen des brevets

(11) Numéro de publication : **0 030 528**
**B1**

(12) # FASCICULE DE BREVET EUROPÉEN

(45) Date de publication du fascicule du brevet : **22.06.83**

(21) Numéro de dépôt : **80870053.8**

(22) Date de dépôt : **05.12.80**

(51) Int. Cl.[3] : **C 07 B 27/00**, C 07 C120/04, C 07 C121/14, C 07 C121/46, C 07 C121/66, C 07 C 53/128, C 07 C 51/353, C 07 C 67/343, C 07 C 69/24, C 07 C102/00, C 07 C103/127

(54) **Procédé pour la fixation de groupes alkyles, aralkyles ou cycloalkyles sur une chaîne carbonée portant un groupe fonctionnel.**

(30) Priorité : **07.12.79 FR 7930039**

(43) Date de publication de la demande : **17.06.81 Bulletin 81/24**

(45) Mention de la délivrance du brevet : **22.06.83 Bulletin 83/25**

(84) Etats contractants désignés : **AT BE CH DE FR IT LI LU NL SE**

(56) Documents cités :
**JOURNAL OF THE CHEMICAL SOCIETY, CHEMICAL COMMUNICATIONS, 1975, London GB L. LALLOZ et al. : « Arynic Synthesis of substituted Indoles », page 745**
**BULLETIN DE LA SOCIETE CHIMIQUE DE FRANCE, no. 11, 1973 Paris FR P. CAUBERE et al. : « Eliminations syn en série halogéno-1-cyclénique », pages 3067-3070**

(73) Titulaire : **SANOFI, société anonyme**
**40, Avenue George V**
**F-75008 Paris (FR)**

(72) Inventeur : **Bouisset, Michel**
**Avenue du 8 mai 1945 Lotissement "Le Thor" No 73**
**F-04200 Sisteron (FR)**
Inventeur : **Chignac, Michel**
**"Le Gand" Avenue du Lac**
**F-04200 Sisteron (FR)**
Inventeur : **Grain, Claude**
**Villa "Les Olivettes" La Croix du Sud**
**F-04290 Volonne (FR)**
Inventeur : **Pigerol, Charles**
**8, rue Carnot**
**F-93400 Saint Ouen (FR)**

(74) Mandataire : **Cauchie, Daniel**
**c/o S.A. LABAZ-SANOFI N.V. Avenue De Béjar, 1**
**B-1120 Bruxelles (BE)**

**EP 0 030 528 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Procédé pour la fixation de groupes alkyles aralkyles ou cycloalkyles sur une chaîne carbonée portant un groupe fonctionnel.

La présente invention concerne un nouveau procédé d'alkylation, le terme « alkylation » étant pris ici dans son sens le plus large, c'est-à-dire englobant, bien qu'improprement d'ailleurs, la fixation, sur une chaîne carbonée, de substituants pouvant être non seulement des radicaux alkyles, linéaires ou ramifiés, mais encore des radicaux aralkyles, linéaires ou ramifiés, mais encore des radicaux aralkyles et cycloalkyles.

Plus précisément, l'invention vise un procédé pour la fixation, par substitution, sur une chaîne carbonée portant un groupement fonctionnel et comportant au moins un proton en position α par rapport à ce groupe fonctionnel, d'au moins un groupe choisi entre les radicaux alkyles, linéaires ou ramifiés, de 1 à 12 atomes de carbone, les radicaux cycloalkyles tels que le cyclohexyle et les radicaux aralkyles tels que benzyle dans lesquels le reste alkyle compte entre 1 et 4 atomes de carbone.

Le groupe fonctionnel précité est un radical nitrile, un radical acide carboxylique, simple ou estérifié par un radical alkyle linéaire ou ramifié de 1 à 5 atomes de carbone, ou encore un radical amide tertiaire de formule générale

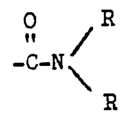

dans laquelle R représente un radical alkyle linéaire ayant de 1 à 3 atomes de carbone.

Ainsi, sous son aspect le plus général, le procédé selon l'invention vise l'obtention de composés de formule générale

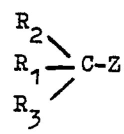

I

dans laquelle $R_1$ représente hydrogène ou un radical alkyle, linéaire ou ramifié, ayant de 1 à 6 atomes de carbone, $R_2$ représente un radical alkyle, linéaire ou ramifié, ayant de 1 à 12 atomes de carbone, un radical aralkyle tel que benzyle ou un radical cycloalkyle tel que cyclohexyle, $R_3$ représente hydrogène ou l'un des radicaux définis pour $R_2$ ci-dessus et Z représente un des groupements fonctionnels cités précédemment, lesdits composés étant obtenus au départ d'une chaîne carbonée de formule générale

$$R_1—CH_2—Z \quad \text{II}$$

dans laquelle $R_1$ et Z ont la même signification que précédemment. Selon une application particulière de ce procédé, l'invention vise la préparation des composés de formule I dans lesquels Z représente un groupe nitrile ou carboxylique et au moins deux des radicaux $R_1$, $R_2$ et $R_3$ représentent un radical propyle, c'est-à-dire, notamment, l'acide di-n-propylacétique, le dipropylacétonitrile et le tripropylacétonitrile. Ces composés et les procédés pour leur préparation sont bien connus. Ainsi, l'acide di-n-propylacétique et ses sels de métaux alcalins sont décrits dans le B.S.M. N° 2.442 M, et largement utilisés pour leurs propriétés neurotropes et particulièrement des propriétés anticonvulsivantes. Le sel de sodium est l'un des anti-épileptiques les plus remarquables sur le marché, et est utilisé également pour le traitement des troubles du caractère ou de la personnalité liés à l'épilepsie.

Le procédé actuellement le plus usuel pour préparer l'acide dipropylacétique est celui décrit au brevet français n° 2 383 907. Ce procédé consiste à traiter le malonate de diéthyle sous pression et en milieu méthanolique, d'abord par du méthylate de sodium, puis par du chlorure d'allyle, selon des conditions opératoires bien définies pour chaque étape.

La diallylmalonate de diéthyle est ensuite saponifié par de la soude et le sel formé est acidifié, pour donner l'acide diallylmalonique, que l'on décarboxyle par chauffage en acide diallylacétique lequel est enfin hydrogéné sur charbon palladié, en acide di-n-propylacétique.

Ce procédé présente l'inconvénient de comporter un nombre relativement élevé d'étapes dans lesquelles les conditions opératoires à respecter engendrent des difficultés d'ordre technique. Au surplus, des réactions secondaires peuvent provoquer la formation d'impuretés qu'il est nécessaire d'éliminer, par exemple la formation d'allyl-2 valérolactone conjointement à l'acide diallylmalonique. Ces inconvénients influencent défavorablement le rendement et le prix de revient du produit final. De même, le dipropylacétonitrile est également connu, et il est utile pour la préparation du dipropylacétamide, qui possède des propriétés neuropsychotropes très précieuses, comme il est indiqué également dans le B.S.M. n° 2 442 M.

Sa préparation est également décrite au brevet français n° 2 383 920 mais selon des processus

compliqués et faisant appel à des réactifs dangereux, tels que le cyanure de sodium.

Enfin, le tripropylacétonitrile est également connu, et utilisé pour la préparation de dérivés de la méthylamine possédant d'intéressantes propriétés pharmacologiques. Ainsi, la tripropylméthylamine est précieuse dans le traitement de la maladie de Parkinson et la correction des troubles axtrapyramidaux, comme décrit au brevet français n° 2 271 811.

Quand on utilise, pour la préparation du tripropylacétonitrile, les procédés classiques de préparation des trialkylacétonitriles par alkylation en position α des nitriles aliphatiques, on obtient des mélanges de nitriles mono-, di- et trisubstitués en α, ainsi que des produits secondaires résultant de la réaction des halogénures d'alkyle sur les nitriles. En d'autres termes, le rendement en produit souhaité et sa pureté sont insuffisants. Au surplus, sa purification par distillation fractionnée dudit mélange est malaisée et abaisse encore le rendement.

C'est aux différents procédés rappelés ci-dessus que l'invention apporte une amélioration, par une simplification résultant de l'abaissement du nombre d'étapes nécessaires et l'abaissement du prix de revient qui en découle.

Le procédé selon l'invention consiste essentiellement, pour fixer, sur une chaîne carbonée de formule II ci-dessus, c'est-à-dire une chaîne portant un groupe fonctionnel et comportant au moins un proton en position α par rapport à ce groupe fonctionnel, au moins un substituant choisi entre les radicaux alkyles de 1 à 12 atomes de carbone, les radicaux cycloalkyles et les radicaux aralkyles dans lesquels le reste alkyle compte de 1 à 4 atomes de carbone :

dans une première étape :

— à faire réagir sur cette chaîne une base complexe constituée par un mélange d'un amidure de métal alcalin et d'un alcoolate de métal alcalin, de manière à donner naissance transitoirement à un carbanion,

puis dans une seconde étape :

— à faire réagir sur ce carbanion un halogénure d'alkyle de formule générale

$$R_2X$$

dans laquelle X représente un atome d'halogène de préférence chlore ou brome et $R_2$ a la même signification que précédemment pour obtenir les composés de formule I dans laquelle $R_3$ représente hydrogène ou un radical identique à $R_2$ et à répéter les deux étapes, avec pour la seconde, la réaction avec un halogénure de formule générale

$$R_2X \text{ ou } R_3X$$

dans laquelle $R_2$ et X ont la même signification que précédemment et $R_3$ a la même signification que précédemment à l'exception d'hydrogène pour obtenir les composés de formule I dans laquelle $R_3$ représente un radical identique à ou différent de $R_2$.

Ainsi donc, dans le cas où l'on veut fixer deux tels groupes substituants sur la chaîne carbonée de formule II, on répète une seconde fois, pour le second substituant les deux mêmes étapes, l'halogénure d'alkyle utilisé dans la seconde étape correspondant, dans les deux couples successifs d'étapes, aux deux groupements à fixer lesquels peuvent être identiques ou différents.

Toutefois, dans le cas où ces deux groupements sont identiques, on peut opérer en une seule fois, pour obtenir directement le produit final, en introduisant un excès de l'halogénure d'alkyle unique utilisé pour assurer la double substitution.

On peut donc schématiquement illustrer le procédé selon l'invention par la succession d'étapes suivantes dans lesquelles $R_3$, qui est différent de l'hydrogène, peut prendre une valeur identique à ou différente de $R_2$.

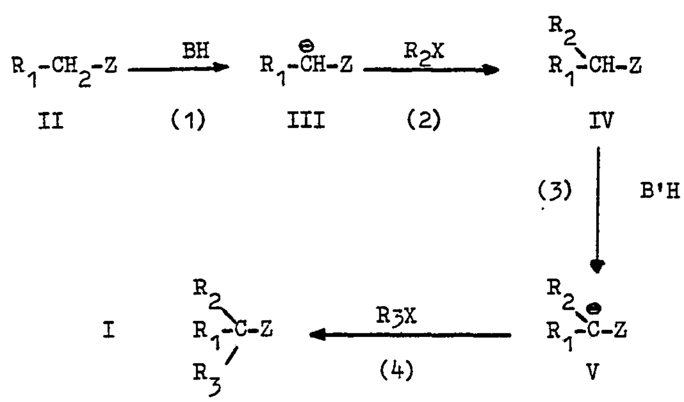


II III IV V

I

Il est clair que, pour chaque couple d'étapes (1)-(2) et (3)-(4), le mécanisme est identique, et que chaque couple peut donc être réalisé indépendamment de l'autre, c'est-à-dire :

— pour l'obtention d'un composé de formule IV, à partir d'un composé de formule II,

— pour l'obtention d'un composé de formule I, à partir d'un composé de formule IV

ou à la suite l'un de l'autre, c'est-à-dire :

— pour l'obtention d'un composé de formule I, à partir d'un composé de formule II, les bases complexes BH et B'H pouvant dans ce cas être identiques ou différentes,

ou enfin, sous forme d'un couple unique répétitif de forme suivante lorsque $R_2$ et $R_3$ ont la valeur de $R_1$ :

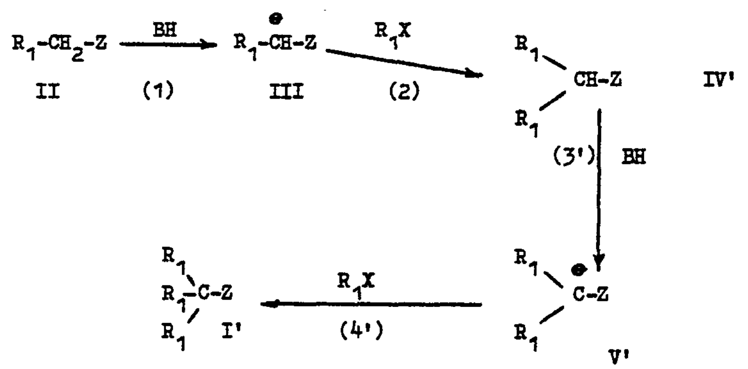


les étapes (3') et (4') étant respectivement identiques aux étapes (1) et (2), en faisant intervenir les mêmes réactifs pour parvenir au composé de formule I' qui est le composé I dans lequel les trois substituants alkyle sont identiques.

C'est le cas où l'on recherche par exemple la formation directe d'un dérivé trialkyle, par exemple le tripropylacétonitrile, comme il est illustré plus loin par des exemples.

Les mélanges désignés ici par « BH » et appelés « bases complexes » sont ceux qui peuvent être représentés par la formule symbolique

$$MNR_4/R_5OM'$$

dans laquelle $R_4$ représente $H_2$, $(C_2H_5)_2$ ou $(iso\text{-}C_3H_7)_2$, $R_5$ représentant un radical aliphatique, linéaire ou ramifié, de 1 à 7 atomes de carbone, ou encore un radical $C_2H_5-O-CH_2-CH_2-$ ou $CH_3-O-CH_2-CH_2-$ et M et M' sont identiques ou différents et représentent un métal alcalin, tel que lithium, sodium ou potassium.

Un tel mélange d'un alcoolate de métal alcalin et d'un amidure alcalin en solution ou en suspension au sein d'un solvant tel que le tétrahydrofuranne, constitue une entité qui a été préparée et étudiée pour la première fois il y a une dizaine d'années, par P. CAUBERE et coll. (voir en particulier Bull. Soc. Chim. F. 1969, p. 2483-9), comme possédant des propriétés basiques extrêmement puissantes.

Ainsi, les auteurs précités ont étudié notamment l'application de telles bases complexes aux alcoylations (voir Bull. Soc. Chim. F. 1971, p. 2334-8) et ont résumé l'ensemble de leurs travaux sur ce sujet dans « Topics in Current Chemistry » 73, Springer-New-York, 1978, p. 49-103.

Il résulte de ce résumé que, s'il est clair que la préparation des carbanions et leur alkylation avec l'aide d'une base complexe représente une réaction de caractère général, l'application de cette réaction à la formation de carbanions dérivés de chaînes renfermant des groupes fonctionnels, telles que définies par la formule I ci-dessus, n'avait encore jamais été envisagée.

La présente invention, qui repose sur cette application, représente un progrès technique considérable, puisqu'elle permet à la fois la simplification du processus d'obtention des composés recherchés, et l'obtention de composés plus purs avec un rendement très élevé.

Sur le plan pratique, d'une manière générale, on forme la base complexe en ajoutant peu à peu une solution de 0,7 mole d'alcool ou d'alcoolate alcalin solide dans le tétrahydrofuranne, à une suspension de 1,4 à 5,6 moles d'amidure alcalin dans un diluant organique anhydre par exemple tétrahydrofuranne, benzène, mélange tétrahydrofuranne/éther isopropylique ou mélange tétrahydrofuranne/benzène. La réaction est exothermique, mais on peut contrôler la température entre 25 et 55 °C, pendant 1 à 2 h. Le procédé selon l'invention, c'est-à-dire chaque couple de réactions (1)-(2) et/ou (3)-(4) ou encore (3')-(4'), consiste à introduire peu à peu la base complexe dans un mélange de 1 mole de composé de départ de formule II ou IV et de 1 mole de l'halogénure d'alkyle de formules $R_2X$ ou $R_3X$ respectivement, en solution dans un solvant organique anhydre, tel que ceux mentionnés aux paragraphes précédents, à une température comprise entre 0 et 72 °C, et de préférence entre 10 et 20 °C, en agitant. On maintient encore le mélange à cette température en agitant pendant 30 min. à 120 min. après la fin de l'introduction de la base complexe. On peut également opérer en inversant l'ordre d'introduction des réactifs, c'est-à-dire introduire la solution des composés de formules II ou IV et de l'halogénure d'alkyle de formules $R_2X$ ou $R_3X$ dans la suspension de base complexe, à une température de − 10 à 20 °C.

À la fin de la réaction, le mélange réactionnel est hydrolysé à une température comprise entre − 10 et

+ 10 °C, pour libérer, après neutralisation ou acidification, selon la nature de Z, le composé de formule I ou I' que l'on recueille par extraction.

Les Exemples suivants illustrent la mise en œuvre de la présente invention, sans pour autant présenter aucun caractère limitatif.

### Exemple 1

Préparation du di-n-propylacétonitrile à partir du valéronitrile

a) Préparation de la base complexe amidure de sodium/t-butylate de sodium dans le rapport 2 : 1

Dans un réacteur de 0,5 l équipé d'un système d'agitation, d'un thermomètre, d'une ampoule d'introduction isobare avec arrivée d'azote et d'un réfrigérant avec une garde à chlorure de calcium, on introduit 150 ml d'éther isopropylique et 100 ml de tétrahydrofuranne. On fait passer de l'azote et on introduit 81,9 g (2,1 moles) d'amidure de sodium en poudre. On introduit dans ce mélange une solution de 51,8 g (0,7 mole) de t-butanol dans 50 ml de tétrahydrofuranne, goutte à goutte sous atmosphère d'azote, et à la température ambiante. La température du milieu réactionnel s'élève à 45-50 °C, et cette température est maintenue constante pendant la fin de l'addition du t-butanol. On maintient encore l'agitation du mélange pendant 1h30 à 45-50 °C, puis on refroidit à 20 °C.

b) Formation du carbanion du valéronitrile et alkylation par le bromure de n-propyle

Dans un réacteur de 1 l muni d'un système d'agitation, d'un thermomètre, d'un réfrigérant avec une garde à chlorure de calcium et d'une ampoule d'introduction isobare, elle-même équipée d'une agitation et d'une arrivée d'azote, on introduit 83 g (1 mole) de valéronitrile, 123 g (1 mole) de bromure de n-propyle et 350 ml d'éther isopropylique.

On place le mélange de base complexe dans l'ampoule d'introduction isobare maintenue sous atmosphère d'azote, et on rince le réacteur deux fois avec 25 ml de tétrahydrofuranne. On place l'ensemble de l'appareillage sous atmosphère d'azote et le mélange de base complexe sous agitation.

On refroidit le mélange renfermé dans le réacteur à 12 ± 1 °C et introduit par fractions le mélange de base complexe, en maintenant la température entre 9 et 18 °C. L'introduction dure 1 h à 1 h 30 ; on maintient encore l'agitation pendant 1 h entre 10 et 15 °C puis on refroidit à 0-5 °C.

On remplace l'ampoule d'introduction de la base complexe par une ampoule identique, chargée avec 100-125 ml d'eau, sous atmosphère d'azote, et on hydrolyse peu à peu le mélange réactionnel à une température inférieure à 10 °C. Le mélange est transvasé dans une ampoule à décantation. On décante la phase aqueuse et on lave la phase organique deux fois avec 125 ml d'eau, deux fois avec 125 ml d'acide chlorhydrique à 10 % et deux fois avec 125 ml d'eau. La phase organique est séchée sur sulfate de sodium. On élimine les solvants par distillation sous pression atmosphérique.

On obtient le di-n-propylacétonitrile avec 81,3 % de rendement.

### Exemple 2

Préparation du di-n-propylacétonitrile à partir du valéronitrile

a) Préparation de la base complexe amidure de sodium/isopropylate de sodium dans le rapport 2 : 1

On opère ainsi qu'il est indiqué au paragraphe a) de l'Exemple 1, mais en utilisant 16,4 g (0,42 mole) d'amidure de sodium, 8,4 g (0,14 mole) d'isopropanol, dans 40 ml d'éther isopropylique et 20 ml de tétrahydrofuranne.

b) Formation du carbanion du valéronitrile et alkylation par le bromure de n-propyle

On opère ainsi qu'il est indiqué au paragraphe b) de l'Exemple 1, sur le mélange de 16,6 g (0,2 mole) de valéronitrile, 24,6 g (0,2 mole) de bromure de n-propyle dans 60 ml d'éther isopropylique.

On introduit le mélange de la base complexe et on rince l'ampoule avec 10 ml de tétrahydrofuranne. L'introduction est faite en 1 h, à une température comprise entre 10 et 15 °C. Après la fin de l'addition on maintient le mélange à 15 °C par 40 à 50 ml d'eau. On traite et on isole le produit de la réaction de la même manière qu'au paragraphe b) de l'Exemple 1.

On évapore les solvants et obtient le di-n-propylacétonitrile avec 62,5 % de rendement.

### Exemple 3

Préparation du di-n-propylacétonitrile à partir du valéronitrile

a) Préparation de la base complexe amidure de sodium/n-propylate de sodium dans le rapport 2 : 1

On opère selon les indications du paragraphe a) de l'Exemple 2, sur les mêmes quantités, mais en utilisant le n-propanol au lieu de l'isopropanol.

b) Formation du carbanion du valéronitrile et alkylation par le bromure de n-propyle

On opère ainsi qu'il est indiqué au paragraphe b) de l'Exemple 2 et on obtient le di-n-propylacétonitrile avec 68,9 % de rendement.

Exemple 4

Préparation du di-n-propylacétonitrile à partir du valéronitrile

a) Préparation de la base complexe amidure de sodium/éthoxy-2 éthylate de sodium

On opère comme il est indiqué au paragraphe a) de l'Exemple 2, mais en employant 12,6 g (0,14 mole) d'éthoxy-2 éthanol au lieu d'isopropanol.

b) Formation du carbanion du valéronitrile et alkylation par le bromure de n-propyle

On opère ainsi qu'il est décrit au paragraphe b) de l'Exemple 2, en effectuant l'addition de la base complexe en 2 h, entre 5 et 10 °C, et on maintient pendant 1 h le mélange entre 10 et 15° après la fin de l'addition. Après traitement on obtient le di-n-propylacétonitrile avec 75,4 % de rendement.

Exemple 5

Préparation du di-n-propylacétonitrile à partir du valéronitrile

a) Préparation de la base complexe amidure de sodium/t-butylate de sodium dans le rapport 3 : 1

On opère ainsi qu'il est indiqué au paragraphe a) de l'Exemple 2, mais en utilisant 21,84 g (0,56 mole) d'amidure de sodium et 12,3 g (0,14 mole) d'alcool t-amylique.

b) Formation du carbanion du valéronitrile et alkylation par le bromure de n-propyle

On opère ainsi qu'il est indiqué au paragraphe b) de l'Exemple 2, sur les mêmes quantités. Après traitement on obtient le di-n-propylacétonitrile avec 59,1 % de rendement.

Exemple 6

Préparation du di-n-propylacétonitrile à partir du valéronitrile

a) Préparation de la base complexe amidure de sodium/t-amylate de sodium dans le rapport 5 : 1

On opère ainsi qu'il est indiqué au paragraphe a) de l'Exemple 2, mais en utilisant 32,76 g (0,84 mole) d'amidure de sodium et 12,3 g (0,14 mole) d'alcool t-amylique.

b) Formation du carbanion du valéronitrile et alkylation par le bromure de n-propyle

On opère ainsi qu'il est indiqué au paragraphe b) de l'Exemple 2, sur les mêmes quantités de valéronitrile et de bromure de n-propyle, mais en maintenant la température entre 0 et 3 °C pendant la durée de l'addition de la base complexe. Après traitement, on obtient le di-n-propylacétonitrile avec 25,1 % de rendement.

Exemple 7

Préparation du di-n-propylacétonitrile à partir du valéronitrile

a) Préparation de la base complexe amidure de sodium/t-butylate de potassium dans le rapport 2 : 1

On prépare la base complexe selon les indications du paragraphe a) de l'Exemple 2, mais en utilisant 10,92 g (0,28 mole) d'amidure de sodium et 15,7 g (0,14 mole) de t-butylate de potassium, et en opérant dans 45 ml de tétrahydrofuranne.

b) Formation du carbanion du valéronitrile et alkylation par le bromure de n-propyle

On opère ainsi qu'il est indiqué au paragraphe b) de l'Exemple 2, sur les mêmes quantités de valéronitrile et de bromure de propyle, mais en utilisant 55 ml de tétrahydrofuranne comme diluant. Après traitement, on obtient le di-n-propylacétonitrile avec 42,5 % de rendement.

Exemple 8

Préparation du di-n-propylacétonitrile à partir du valéronitrile

a) Préparation de la base complexe amidure de lithium/t-butylate de potassium dans le rapport 2 : 1

On introduit 0,97 g (0,14 mole) de lithium dans 200 ml d'ammoniac liquide en opérant entre − 40 et 45 °C et on maintient le mélange sous agitation pendant une nuit. On chasse ensuite l'ammoniac liquide et on introduit 7,9 g (0,07 mole) de t-butylate de potassium et 35 ml de tétrahydrofuranne. On chauffe le mélange pendant 2 heures sous agitation à 55 °C.

b) Formation du carbanion du valéronitrile et alkylation par le bromure de n-propyle

On refroidit le mélange préparé selon a) à 15 °C et on introduit goutte à goutte une solution de 8,3 g (0,1 mole) de valéronitrile, 12,3 g (0,1 mole) de bromure de n-propyle dans 30 ml de tétrahydrofuranne. On effectue cette addition en 1 h, à une température de 15 °C, et on maintient le mélange pendant 1 h à cette température après la fin de l'addition. On traite le produit de la réaction de la manière habituelle et on obtient le di-n-propylacétonitrile avec 56,4 % de rendement.

Exemple 9

Préparation du di-n-propylacétonitrile à partir du valéronitrile

a) Préparation de la base complexe diéthylamidure de lithium/t-amylate de lithium dans le rapport 2 : 1

On introduit une solution de 6,15 g (0,07 mole) d'alcool t-amylique dans 30 ml de tétrahydrofuranne dans 160 ml d'une suspension de diéthylamidure de lithium à 15 % dans l'hexane (0,21 mole) et on chauffe le mélange sous agitation pendant 2 h à 55 °C. En fin de réaction, la base complexe forme une solution.

b) Formation du carbanion du valéronitrile et alkylation par le bromure de n-propyle

On opère ainsi qu'il est indiqué au paragraphe b) de l'Exemple 8, sur les mêmes quantités de bromure de n-propyle et de valéronitrile (soit 0,1 mole). On traite le produit de la réaction de la lumière habituelle et on obtient le di-n-propylacétonitrile avec 35 % de rendement.

Exemple 10

Préparation du di-n-propylacétonitrile à partir du valéronitrile

a) Préparation de la base complexe amidure de sodium/t-butylate de sodium dans le rapport 2 : 1

On opère ainsi qu'il est indiqué au paragraphe a) de l'Exemple 2, avec les mêmes quantités de réactifs, mais en utilisant le t-butanol, 10,4 g (0,14 mole), à la place de l'isopropanol et le système de diluants benzène 30 ml et tétrahydrofuranne 30 ml.

b) Formation du carbanion du valéronitrile et alkylation par le bromure de n-propyle

On opère ainsi qu'il est indiqué au paragraphe b) de l'Exemple 2, mais en utilisant 65 ml de benzène et 65 ml de tétrahydrofuranne, à une température comprise entre 14 et 18 °C. On laisse encore 1 h à 15 °C après la fin de l'addition. On isole le produit selon la méthode habituelle et obtient le di-n-propylacétonitrile avec 41,5 % de rendement.

Exemple 11

Préparation du di-n-propylacétonitrile à partir du valéronitrile

a) Préparation de la base complexe amidure de sodium/t-amylate de sodium dans le rapport 2 : 1

On opère ainsi qu'il est indiqué au paragraphe a) de l'Exemple 2, mais en utilisant 12,3 g (0,14 mole)

d'alcool t-amylique dans 30 ml de benzène au lieu d'isopropanol et 16,4 g d'amidure de sodium (0,42 mole) dans 30 ml de benzène.

b) Formation du carbanion du valéronitrile et alkylation par le bromure de n-propyle

On opère ainsi qu'il est indiqué au paragraphe b) de l'Exemple 2 avec les mêmes quantités de valéronitrile et de bromure de n-propyle dans 160 ml de benzène. L'introduction de la base complexe à laquelle on a ajouté 34 ml d'hexaméthylphosphorotriamide dans le mélange est effectuée entre 5 et 13 °C, et après avoir maintenu l'agitation à cette température pendant 2 h après la fin de l'addition on traite de la manière habituele et on obtient le di-n-propylacétonitrile avec 36,6 % de rendement.

Exemple 12

Préparation du di-n-propylacétonitrile à partir du valéronitrile

a) Préparation de la base complexe amidure de sodium/t-amylate de sodium dans le rapport 2 : 1

On prépare la base complexe ainsi qu'il est indiqué au paragraphe a) de l'Exemple 2, mais à partir de 12,3 g (0,14 mole) d'alcool t-amylique au lieu de l'isopropanol et en employant 70 ml de tétrahydrofuranne comme diluant.

b) Formation du carbanion du valéronitrile et alkylation par le bromure de n-propyle

On opère ainsi qu'il est indiqué au paragraphe b) de l'Exemple 2 avec les mêmes quantités de bromure de n-propyle et de valéronitrile dans 60 ml de tétrahydrofuranne mais l'addition de la base complexe dans le mélange est effectuée à la température de reflux du mélange. Après la fin de l'addition, on maintient encore la température du mélange à 72 °C pendant 2 h. On traite le produit de la réaction de la manière habituelle et on obtient le di-n-propylacétonitrile avec 52 % de rendement.

Exemple 13

Préparation du di-n-propylacétonitrile à partir du valéronitrile

a) Préparation de la base complexe amidure de sodium/t-butylate de sodium dans le rapport 2 : 1

On prépare la base complexe ainsi qu'il est indiqué dans le paragraphe a) de l'Exemple 2, à partir de 4,87 g d'amidure de sodium (0,125 mole), et 3,14 g (0,042 5 mole) d'alcool t-butylique, en employant 80 ml de tétrahydrofuranne comme diluant, mais sans laisser la température s'élever au-dessus de 25 °C, et sans chauffer le mélange de base complexe.

b) Formation du carbanion du valéronitrile et alkylation par le bromure de n-propyle

On dissout 8,3 g (0,1 mole) de valéronitrile et 12,3 g (0,1 mole) de bromure de n-propyle dans 70 ml de tétrahydrofuranne. On refroidit à 1 °C le mélange préparé selon a) et on introduit la solution de valéronitrile et de bromure de n-propyle dans le mélange de la base complexe, à une vitesse telle que la température ne dépasse pas 4 °C. L'addition dure 45 minutes. On laisse remonter la température vers 20 °C, en 1 heure, et on hydrolyse de la manière habituelle. On obtient le di-n-propylacétonitrile avec 51 % de rendement.

Exemple 14

Préparation du n-propyl-2-tétradécanenitrile à partir du valéronitrile

a) Préparation de la base complexe amidure de sodium/t-amylate de sodium dans le rapport 2 : 1

On prépare la base complexe selon les indications du paragraphe a) de l'Exemple 2, mais à partir de 16,4 g (0,42 mole) d'amidure de sodium et de 12,3 g (0,14 mole) d'alcool t-amylique, et en employant 70 ml de tétrahydrofuranne comme diluant.

b) Formation du carbanion du valéronitrile et alkylation par le chloro-1 dodécane

On opère ainsi qu'il est indiqué au paragraphe b) de l'Exemple 2, mais en utilisant 16,6 g (0,2 mole) de valéronitrile et 40,95 g (0,2 mole) de chloro-1 dodécane, dans 60 ml de tétrahydrofuranne et on obtient le n-propyl-2 tétradécanenitrile avec 24,6 % de rendement.

Exemple 15

Préparation de l'α-n-propyl-α-cyclohexyl-acétonitrile à partir du valéronitrile

a) Préparation de la base complexe amidure de sodium/t-amylate de sodium dans le rapport 2 : 1

On opère ainsi qu'il est indiqué au paragraphe a) de l'Exemple 14, et sur les mêmes quantités de réactifs et diluant.

b) Formation du carbanion du valéronitrile et alkylation par le chlorure de cyclohexyle

On opère ainsi qu'il est indiqué au paragraphe b) de l'Exemple 2, mais en utilisant 16,6 g (0,2 mole) de valéronitrile et 23,7 g (0,2 mole) de chlorure de cyclohexyle, dans 60 ml de tétrahydrofuranne, et on obtient l'α-n-propyl-α-cyclohexyl-acétonitrile avec 26,3 % de rendement.

Exemple 16

Préparation de l'α-n-propyl-α-benzyl-propionitrile à partir du valéronitrile

a) Préparation de la base complexe amidure de sodium/t-amylate de sodium dans le rapport 2 : 1

On opère ainsi qu'il est indiqué au paragraphe a) de l'Exemple 14, et sur les mêmes quantités de réactifs et de diluant.

b) Formation du carbanion du valéronitrile et alkylation par le chlorure de benzyle

On opère ainsi qu'il est indiqué au paragraphe b) de l'Exemple 2, mais en utilisant 16,6 g (0,2 mole) de valéronitrile et 25,3 g (0,2 mole) de chlorure de benzyle, dans 60 ml de tétrahydrofuranne et on obtient l'α-n-propyl-α-benzyl-propionitrile avec 52,8 % de rendement.

Exemple 17

Préparation du méthyl-4-n-propyl-2 pentanenitrile

a) Préparation de la base complexe amidure de sodium/t-amylate de sodium dans le rapport 2 : 1

On opère ainsi qu'il est indiqué au paragraphe a) de l'Exemple 14 et sur les mêmes quantités de réactifs et de diluant.

b) Formation du carbanion du valéronitrile et alkylation par le bromure d'isobutyle

On opère ainsi qu'il est indiqué au paragraphe b) de l'Exemple 2, mais en utilisant 16,6 g (0,2 mole) de valéronitrile et 27,4 g (0,2 mole) de bromure d'isobutyle, et on obtient le méthyl-4 n-propyl-2 pentanenitrile avec 63 % de rendement.

Exemple 18

Préparation du di-n-propylacétonitrile à partir de l'acétonitrile

a) Préparation de la base complexe amidure de sodium/t-butylate de sodium dans le rapport 2 : 1

On prépare la base complexe selon les indications du paragraphe a) de l'Exemple 1, à partir de 164 g (4,2 moles) d'amidure de sodium dans 500 ml de tétrahydrofuranne et 103,6 g (1,4 mole) de t-butanol dans 700 ml de tétrahydrofuranne.

b) Formation du carbanion de l'acétonitrile et alkylation par le bromure de n-propyle

On suit les indications du paragraphe b) de l'Exemple 1 en effectuant la réaction sur un mélange de 41 g (1 mole) d'acétonitrile et 246 g (2 moles) de bromure de n-propyle en solution dans 600 ml de tétrahydrofuranne, le mélange étant refroidi à 0 °C. L'addition de la suspension de la base complexe est effectuée sur une durée de 1 h 30 à 2 h, pendant laquelle la température du mélange réactionnel est maintenue entre 0 et 5 °C. On laisse ensuite la température remonter à 10 °C et on maintient le mélange à cette température pendant 1 h sous agitation et sous atmosphère d'azote. On hydrolyse le mélange en le versant lentement dans un mélange de 400 ml d'eau et de 400 ml d'éther sous agitation. On décante la phase aqueuse et on concentre la solution organique sous vide. On obtient le di-n-propylacétonitrile avec 83,7 % de rendement.

Exemple 19

Préparation du di-n-propylacétonitrile à partir de l'acétonitrile

a) Préparation de la base complexe amidure de sodium/t-butylate de sodium dans le rapport 2 : 1

On opère ainsi qu'il est indiqué sous le paragraphe a) de l'Exemple 2, mais à partir de 8,2 g (0,21 mole) d'amidure de sodium et 5,2 g (0,07 mole) de t-butanol, et dans 80 ml de tétrahydrofuranne.

b) Formation du carbanion de l'acétonitrile et alkylation par le bromure de n-propyle

On opère ainsi qu'il est indiqué au paragraphe b) de l'Exemple 13, mais en utilisant 4,1 g (0,1 mole) d'acétonitrile et 24,6 g (0,02 mole) de bromure de n-propyle en solution dans 70 ml de tétrahydrofuranne. Le mélange de la base complexe est refroidi à − 10 °C avant d'être traité peu à peu par le mélange d'acétonitrile et de bromure de n-propyle, à cette température et en 40 minutes. Après le traitement habituel on obtient le di-n-propylacétonitrile avec 62,8 % de rendement.

Exemple 20

Préparation du dibenzylacétonitrile à partir de l'acétonitrile

a) Préparation de la base complexe amidure de sodium/t-amylate de sodium dans le rapport 2 : 1

On prépare la base complexe selon les indications du paragraphe a) de l'Exemple 14, sur les mêmes quantités de réactifs et de diluant.

b) Formation du carbanion de l'acétonitrile et alkylation par le chlorure de benzyle

On suit les indications du paragraphe b) de l'Exemple 18, mais en effectuant la réaction sur 4,1 g (0,1 mole) d'acétonitrile et 25,3 g (0,2 mole) de chlorure de benzyle en solution dans 30 ml de tétrahydrofuranne. On obtient le dibenzylacétonitrile avec 75,3 % de rendement.

Exemple 21

Préparation du diisobutylacétonitrile à partir de l'acétonitrile

a) Préparation de la base complexe amidure de sodium/t-amylate de sodium dans le rapport 2 : 1

On prépare la base complexe selon les indications du paragraphe a) de l'Exemple 14, sur les mêmes quantités de réactifs et de diluant.

b) Formation du carbanion de l'acétonitrile et alkylation par le bromure d'isobutyle

On suit les indications du paragraphe b) de l'Exemple 18, mais en effectuant la réaction sur 4,1 g (0,1 mole) d'acétonitrile et 27,4 g (0,2 mole) de bromure d'isobutyle en solution dans 30 ml de tétrahydrofuranne. On obtient le diisobutylacétonitrile avec 45 % de rendement.

Exemple 22

Préparation du tri-n-propylacétonitrile à partir de l'acétonitrile

a) Préparation de la base complexe amidure de sodium/t-butylate de sodium dans le rapport 2 : 1

On prépare la base complexe selon le paragraphe a) de l'Exemple 2 mais à partir de 17,55 g (0,45 mole) d'amidure de sodium et 11,10 g (0,15 mole) de t-butanol dans 40 ml de tétrahydrofuranne.

b) Formation du carbanion d'acétonitrile et alkylation par le bromure de n-propyle

On ajoute au mélange de base complexe préparée sous a) un mélange de 4,1 g (0,1 mole) d'acétonitrile et 40,59 g (0,33 mole) de bromure de n-propyle. La durée de l'addition est de 25 minutes durant laquelle la température est maintenue entre 8 et 26 °C.

Après l'addition, le mélange est encore maintenu sous agitation durant 1 heure à température ambiante. Après le traitement habituel, on obtient le tri-n-propylacétonitrile avec 66 % de rendement.

Exemple 23

Préparation du tri-n-propylacétonitrile à partir du di-n-propylacétonitrile

a) Préparation de la base complexe amidure de sodium/t-butylate de sodium dans le rapport 2 : 1

On prépare la base complexe selon le paragraphe a) de l'Exemple 2, mais à partir de 8,2 g (0,21 mole) d'amidure de sodium et de 5,2 g (0,07 mole) de t-butanol dans 100 ml de tétrahydrofuranne.

b) Formation du carbanion du di-n-propylacétonitrile et alkylation par le bromure de n-propyle

On ajoute au mélange de base complexe préparée sous a) et maintenu à 20 °C ± 2 °C, le mélange de 12,5 g (0,1 mole) de di-n-propylacétonitrile et 16 g (0,13 mole) de bromure de n-propyle. La durée de l'addition est de 5 minutes. Le mélange est gardé sous agitation et sa température est maintenu entre 20 et 30 °C pendant 40 minutes, après lesquelles elle revient lentement à 24 °C. L'hydrolyse est effectuée de la manière habituelle. Après traitement on obtient le tri-n-propylacétonitrile avec 94,5 % de rendement.

Exemple 24

Préparation de l'acide di-n-propylacétique à partir de l'acide acétique

a) Préparation de la base complexe amidure de sodium/t-butylate de sodium dans le rapport 2 : 1

On met en suspension dans un erlenmeyer 23,4 g (0,6 mole) d'amidure de sodium dans 60 ml de tétrahydrofuranne en opérant sous atmosphère d'azote. On introduit une solution de 14,8 g (0,2 mole) de t-butanol dans 10 ml de tétrahydrofuranne. La température s'élève à 50 °C. On maintient la température du mélange à 55 °C pendant 1 h 30 puis on laisse redescendre à la température ambiante.

b) Formation du carbanion de l'acide acétique et alkylation par le bromure de n-propyle

Dans un erlenmeyer on introduit 4,8 g (0,1 mole) d'une dispersion d'hydrure de sodium à 50 % dans une huile minérale et 60 ml de tétrahydrofuranne, en opérant sous atmosphère d'azote. On introduit ensuite goutte à goutte une solution de 6 g (0,1 mole) d'acide acétique et 10 ml de tétrahydrofuranne. La température s'élève à 55-60 °C. On maintient sous agitation pendant 2 h le gel d'acétate de sodium formé. On introduit ensuite, à 20 °C, en une seule fois la base complexe préparée sous a) et on rince avec 10 ml de tétrahydrofuranne l'erlenmeyer. On chauffe le mélange à 50 °C pendant 1 h, puis on introduit dans le mélange gardé à 50-55 °C, 24,6 g (0,2 mole) de bromure de n-propyle. A la fin de l'addition du bromure de n-propyle, la température atteint 60 °C ; on maintient ensuite pendant 1 h la température du mélange à 55 °C puis on laisse le mélange pendant une nuit à 20 °C. On hydrolyse le mélange par addition d'eau. On décante la couche aqueuse qui est acidifiée par addition d'une solution d'acide chlorhydrique concentré, puis on extrait plusieurs fois avec de l'éther éthylique. On lave la phase éthérée avec de l'eau, on la sèche et on évapore l'éther. On obtient avec 63,6 % de rendement un mélange renfermant 18,6 % d'acide di-n-propylacétique et 45 % d'acide valérique.

Exemple 25

Préparation de l'acide di-n-propylacétique à partir de l'acide valérique

a) Préparation de la base complexe amidure de sodium/t-butylate de sodium dans le rapport 2 : 1

On prépare la base complexe ainsi qu'il est indiqué au paragraphe a) de l'Exemple 21, en utilisant 11,7 g (0,3 mole) d'amidure de sodium, 7,4 g (0,1 mole) de t-butanol dans 80 ml de tétrahydrofuranne.

b) Formation du carbanion de l'acide valérique et alkylation par le bromure de n-propyle

On prépare le valérate de sodium ainsi qu'il est indiqué au paragraphe b) de l'Exemple 21 pour l'acétate de sodium mais en employant 4,8 g (0,1 mole) de dispersion d'hydrure de sodium à 50 % dans une huile minérale, 60 ml de tétrahydrofuranne et 10,2 g (0,1 mole) d'acide valérique.

On introduit ensuite 18,45 g (0,15 mole) de bromure de n-propyle et on chauffe ensemble à 60 °C, toujours sous atmosphère d'azote. On ajoute au mélange placé sous agitation la base complexe, peu à peu, en 1 h 20 min. On rince l'ampoule d'introduction avec 20 ml de tétrahydrofuranne. On maintient encore l'ensemble pendant 1 h 30 à 60 °C et on laisse une nuit à 20 °C. On hydrolyse et on traite comme au paragraphe b) de l'Exemple 21. On obtient avec un rendement quantitatif un mélange renfermant 83,3 % d'acide valérique et 16,7 d'acide di-n-propylacétique.

Exemple 26

Préparation de l'acide di-n-propylacétique à partir de l'acide valérique

a) Préparation de la base complexe amidure de lithium/t-amylate de lithium dans le rapport 2 : 1

On prépare l'amidure de lithium par dissolution de 1 g (0,15 mole) de lithium laminé dans 200 ml d'ammoniac liquide en présence de quelques cristaux de nitrate ferrique comme catalyseur, en opérant vers – 40 à – 45 °C. Après la fin de réaction, on chasse l'ammoniac et on prépare sous atmosphère d'azote la base complexe en introduisant 8,8 g (0,1 mole) d'alcool t-amylique en solution dans 30 ml de tétrahydrofuranne. On chauffe le mélange pendant 2 h à 55-60 °C.

b) Formation du carbanion de l'acide valérique et alkylation par le bromure de n-propyle

On prépare le valérate de lithium sous atmosphère d'azote en introduisant 33 ml (0,055 mole) d'une solution à 15 % de butyllithium dans l'hexane dans une solution de 5,1 g (0,05 mole) d'acide valérique dans 30 ml de tétrahydrofuranne. On chauffe le mélange pendant 1 h à 60 °C. On introduit ensuite la base complexe dans la suspension de valérate de lithium et on chauffe ensuite le mélange pendant 1 h à 60 °C. On ajoute alors 9,25 g (0,075 mole) de bromure de n-propyle en 1 heure et on chauffe le mélange à 60 °C pendant 1 h 30. On traite le mélange comme il est indiqué à la fin du paragraphe b) de l'exemple 21. On obtient avec 72,5 % de rendement un mélange renfermant 21,5 % d'acide di-n-propylacétique et 51 % d'acide valérique.

Exemple 27

Préparation de l'acide di-n-propylacétique à partir de l'acide valérique

a) Préparation de la base complexe diéthylamidure de lithium/t-butylate de lithium dans le rapport 2 : 1

On ajoute peu à peu 3,2 g (0,46 mole) de lithium en copeaux à un mélange de 13,2 g (0,15 mole) de t-butanol et de 21,9 g (0,3 mole) de diéthylamine dans 54 ml de benzène et 54 ml d'hexaméthylphosphorotriamide. On maintient le mélange réactionnel sous agitation pendant 5 h entre 25 et 30 °C.

b) Formation du carbanion de l'acide valérique et alkylation par le bromure de n-propyle

On prépare du valérate de lithium selon les indications du paragraphe b) de l'Exemple 26, dans le tétrahydrofuranne, mais en isolant le valérate de lithium par évaporation du tétrahydrofuranne.

On met en suspension 16,2 g (0,15 mole) de valérate de lithium dans un mélange de 90 ml d'hexane et de 90 ml de toluène, et on ajoute le mélange de base complexe préparé sous a) en maintenant la température du milieu réactionnel à 20 °C. Après la fin de l'addition de la base complexe, on porte la température du mélange à 36 °C pendant 1 heure. On ajoute alors 27,54 g (0,22 mole) de bromure de n-propyle. La température du mélange s'élève à 50 °C. On maintient la température du mélange entre 50 et 57 °C pendant une nuit. On refroidit le mélange à – 5 °C et on introduit 100 ml d'acide chlorhydrique concentré en ne laissant pas la température dépasser 0 °C. On élimine les solvants sous pression réduite à une température n'excédant pas 40 °C et on dilue par addition de 75 ml d'eau. On extrait le mélange 4 fois avec 250 ml d'éther. Les phases éthérées sont réunies. On lave la solution éthérée avec de l'eau. On la sèche sur sulfate de sodium et on élimine l'éther sous pression réduite.

On obtient l'acide di-n-propylacétique avec 35,8 % de rendement et on récupère 50,7 % d'acide valérique.

Exemple 28

Préparation du di-n-propylacétate de t-butyle à partir du valérate de t-butyle

a) Préparation de la base complexe amidure de sodium/t-amylate de sodium dans le rapport 2 : 1

On opère ainsi qu'il est indiqué au paragraphe a) de l'Exemple 2, mais en utilisant 6 g (0,155 mole) d'amidure de sodium dans 25 ml de tétrahydrofuranne et 3,1 g (0,035 mole) d'alcool t-amylique dans 10 ml de tétrahydrofuranne.

b) Formation du carbanion du valérate de t-butyle et alkylation par le bromure de n-propyle

On refroidit à – 10 °C le mélange préparé sous a) et on ajoute peu à peu une solution de 7,9 g (0,05 mole) de valérate de t-butyle dans 10 ml de tétrahydrofuranne. On maintient encore pendant 1 heure

sous agitation, à – 10 °C après la fin de l'addition. On ajoute alors, toujours à – 10 °C, 7,4 g (0,06 mole) de bromure de n-propyle et 9 g (0,05 mole) d'hexaméthylphosphorotriamide. On maintient le mélange pendant 1 heure sous agitation vers – 10 °C.

On hydrolyse par addition d'eau et on extrait à l'éther. Après séchage de la phase éthérée, on évapore l'éther. On obtient le di-n-propylacétate de t-butyle avec 49,5 % de rendement.

Exemple 29

Préparation du NN-diéthyl di-n-propylacétamide à partir du NN-diéthyl valéramide

a) Préparation de la base complexe amidure de sodium/t-amylate de sodium dans le rapport 2 : 1

On prépare la base complexe ainsi qu'il est indiqué au paragraphe a) de l'Exemple 2, mais en utilisant 8,2 g (0,21 mole) d'amidure de sodium et 6,16 g (0,07 mole) d'alcool t-amylique, et en opérant dans 50 ml de tétrahydrofuranne.

b) Formation du carbanion du NN-diéthyl valéramide et alkylation par le bromure de n-propyle

On refroidit un mélange de 15,7 g (0,1 mole) de NN-diéthyl valéramide et de 12,3 g (0,1 mole) de bromure de n-propyle dans 20 ml de tétrahydrofuranne à 2 °C et on y ajoute le mélange de la base complexe en agitant et en ne laissant pas la température dépasser 20 °C. On laisse encore l'agitation pendant 1 h 30 à cette température et on hydrolyse de la manière habituelle. On obtient le NN-diéthyl di-n-propylacétamide avec 27,75 % de rendement. On récupère 46,75 % de NN-diéthyl valéramide inchangé.

Exemple 30

Préparation de NN-diéthyl di-n-propylacétamide à partir du NN-diéthyl valéramide

a) Préparation de la base complexe amidure de sodium t-amylate de sodium dans le rapport 2 : 1

On opère ainsi qu'il est indiqué au paragraphe a) de l'Exemple 28 mais en employant 5 ml de tétrahydrofuranne.

b) Formation du carbanion du NN-diéthyl valéramide et alkylation par le bromure de n-propyle

On ajoute le mélange de base complexe goutte à goutte dans la solution de 15,7 g (0,1 mole) de NN-diéthyl valéramide dans 15 ml d'éther isopropylique, en opérant sous azote, à 20 °C. On laisse le mélange sous agitation à la température ambiante pendant 1 h, puis on introduit 12,3 g (0,1 mole) de bromure de n-propyle en solution dans 15 ml d'éther isopropylique et on maintient encore l'agitation pendant 45 minutes. On hydrolyse et on isole de la manière habituelle. On obtient le NN-diéthyl di-n-propylacétamide avec 34,9 % de rendement. On récupère 55 % de NN-diéthyl valéramide inchangé.

Exemple 31

Préparation du valéronitrile à partir de l'acétonitrile

a) Préparation de la base complexe amidure de sodium éthoxy-2 éthylate de sodium

Dans un ballon de 500 ml, parfaitement sec, on introduit 62,5 g d'amidure de sodium et 145 g (200 ml) d'éther diisopropylique. Sous agitation, à température ambiante sous atmosphère d'azote, on ajoute, goutte à goutte, une solution de 48,02 g d'éthoxy-2 éthanol et 72,5 g (100 ml) d'éther diisopropylique tout en maintenant la température à 45-50 °C. On poursuit l'agitation durant 3 heures supplémentaires sous atmosphère d'azote et à 60 °C puis pendant 8 heures à la température ambiante.

b) Formation du carbanion de l'acétonitrile et alkylation avec le bromure de n-propyle

Dans un ballon de 1 l, parfaitement sec, on introduit 53,5 g d'acétonitrile, 123,0 g de bromure de n-propyle et 289,5 g (400 ml) d'éther diisopropylique. On agite le milieu à la température ambiante tout en le maintenant sous atmosphère d'azote. On transfère la suspension de base complexe dans une ampoule d'introduction de 500 ml et on maintient l'agitation. On rince le flacon qui a contenu la base complexe avec 72,5 g (100 ml) d'éther diisopropylique et on ajoute cet éther au contenu de l'ampoule d'introduction. On ajoute, par fractions, la base complexe au milieu réactionnel tout en maintenant la température du mélange entre 30 et 35 °C. Cette opération nécessite 30 minutes. On abandonne alors le milieu pendant 30 minutes puis on refroidit à 0 à 5 °C. Aorès hydrolyse à cette température par ajout progressif de 160 g d'eau distillée, on décante la phase aqueuse pendant 15 minutes. On lave la phase organique

avec successivement 65 g d'eau distillée, 47 g d'acide chlorhydrique à 36 % et 3 fractions, chacune de 125 g d'eau distillée. On sèche la phase organique sur sulfate de sodium et on évapore le solvant à pression atmosphérique pour atteindre 73 ± 1 °C en tête de colonne.

De cette manière, on obtient 63,5 g d'une huile brute titrant 88,2 % en produit désiré.

Rendement en valéronitrile : 67,5 %.

En utilisant la même méthode que celle décrite ci-dessus, on a préparé les composés suivants au départ des produits appropriés :

Composés

Hydrocinnamonitrile à partir du bromure de benzyle
Le produit brut obtenu titre 62,1 % en produit désiré
Rendement : 64,8 %

Isocapronitrile à partir du bromure d'isobutyle
Le produit brut obtenu titre 81,3 % en produit désiré
Rendement : 35,5 %

Penténo-4 nitrile à partir de bromure d'allyle
Le produit brut obtenu titre 58 % en produit désiré
Rendement : 53,7 %

Exemple 32

Préparation du diisobutylacétonitrile à partir de l'acétonitrile

a) Préparation de la base complexe amidure de sodium éthoxy-2 éthylate de sodium

On opère ainsi qu'il est indiqué au paragraphe a) de l'Exemple 30, sur le mélange de 66,6 g d'amidure de sodium dans 181 g (250 ml) d'éther diisopropylique et 51 g d'éthoxy-2 éthanol dans 54,5 g (75 ml) d'éther diisopropylique.

b) Formation du carbanion de l'acétonitrile et alkylation par le bromure d'isobutyle

Dans un ballon, on introduit 20,5 g d'acétonitrile, 137 g de bromure d'isobutyle et 325,5 g (450 ml) d'éther diisopropylique. On effectue la réaction à 30 °C ± 2°, l'introduction de la base complexe nécessitant 40 minutes. Les conditions opératoires de réaction et d'isolation sont les mêmes que celles décrites au paragraphe b) de l'Exemple 30.

De cette manière, on obtient 59,75 g d'une huile brute titrant 76,4 % en produit désiré.

Rendement en diisobutylacétonitrile : 60 %

En utilisant la même méthode que celle décrite précédemment on a préparé le diallylacétonitrile à partir du bromure d'allyle.

Le produit brut obtenu titre 39,2 % en composé désiré.

Rendement en diallylacétonitrile : 30,7 %.

Exemple 33

Préparation du dibenzylacétonitrile à partir de l'acétonitrile

a) Préparation de la base complexe amidure de sodium t-amylate de sodium

Dans un ballon de 500 ml, parfaitement sec, on introduit 81,9 g (2,1 moles) d'amidure de sodium en poudre et 88,8 g (100 ml) de tétrahydrofuranne sec. A température ambiante, sous agitation et sous atmosphère d'azote, on ajoute, goutte à goutte, une solution de 61,6 g (0,7 mole) d'alcool t-amylique et 44,4 g (50 ml) de tétrahydrofuranne tout en maintenant la température entre 45 et 50 °C.

On maintient le milieu à 60 °C pendant 90 minutes puis pendant 1 heure à température ambiante.

b) Formation du carbanion de l'acétonitrile et alkylation par le chlorure de benzyle

Dans un ballon de 1 l, parfaitement sec, on introduit 20,5 g (0,5 mole) d'acétonitrile sec, 126,5 g (1 mole) de chlorure de benzyle et 266,4 g (300 ml) de tétrahydrofuranne sec. On agite sous atmosphère d'azote tout en maintenant la température de masse à 10/15 °C durant l'opération d'introduction de la suspension de base complexe.

On maintient le milieu à 15 °C pendant 1 heure après l'introduction et on hydrolyse, à température n'excédant pas 15 °C, par ajout d'un mélange de 400 g d'eau distillée et 285,6 g (400 ml) d'éther

éthylique. On décante la phase aqueuse et on lave la phase organique successivement avec une solution de 70 g d'eau et 50 g d'acide chlorhydrique à 36 % puis avec 3 fractions chacune de 120 g d'eau. On sèche sur sulfate de sodium et on élimine le solvant sous pression atmosphérique jusqu'à atteindre 80 °C en tête de colonne.

De cette manière, on obtient 115 g d'une huile brute titrant 63,6 % en produit désiré.

Rendement en dibenzylacétonitrile : 66,2 %.

Exemple 34

Préparation de l'éthyl-2 valéronitrile à partir du valéronitrile

a) Préparation de la base complexe amidure de sodium/éthoxy-2 éthylate de sodium

On opère ainsi qu'il est indiqué au paragraphe a) de l'Exemple 31.

b) Formation du carbanion du valéronitrile et alkylation par le bromure d'éthyle

On opère ainsi qu'il est indiqué au paragraphe b) de l'Exemple 31 mais en effectuant l'alkylation entre 28 et 30 °C tout en maintenant le milieu pendant 1 heure à température ambiante avant hydrolyse.

De cette manière, on obtient 114 g d'une huile brute titrant 77,7 % en produit désiré.

Rendement en éthyl-2 valéronitrile : 80 %.

Suivant le même procédé que celui décrit précédemment mais au départ des produits appropriés, on a préparé les composés suivants en tenant compte des modifications indiquées :

Composés

Allyl-2 valéronitrile
Durée de l'opération d'addition de la base complexe : 40 min.
Température d'alkylation : 30 à 35 °C
L'huile brute obtenue titre 60,8 % en produit désiré
Rendement : 61,3 %.

Isobutyl-2 valéronitrile
Durée de l'opération d'addition de la base complexe : 27 min.
Température d'alkylation : 35 à 38 °C
L'huile brute obtenue titre 97,3 % en produit désiré
Rendement : 72,7 %
P.E. : 190 °C ou 99-101 °C sous 20 mm Hg
$n_D^{20} = 1{,}419\,9$

Spectre I.R. : $C \equiv N$ à $\simeq$ 2 245 $cm^{-1}$
Benzyl-2 valéronitrile
Durée de l'opération d'addition de la base complexe : 45 min.
Température d'alkylation : 30 à 35 °C
L'huile brute obtenue titre 98,9 % en produit désiré.
Rendement : 62,5 %
P.E. : 85-86 °C sous 0,2 mm Hg
$n_D^{20} = 1{,}504\,8$

Spectre I.R. : $C \equiv N$ à $\simeq$ 2 245 $cm^{-1}$
Dodécyl-2 valéronitrile
Durée de l'opération d'introduction de la base complexe : 20 min.
Température d'alkylation : 45 à 50 °C
L'huile brute obtenue titre 99,5 % en produit désiré
Rendement : 52,4 %
P.E. : 140 °C sous 0,4 mm Hg ou 127 °C sous 0,15 mm Hg
$n_D^{24} = 1{,}442\,5$

Spectre I.R. : $C \equiv N$ à $\simeq$ 2 245 $cm^{-1}$ et 2 195 $cm^{-1}$
Propargyl-2 valéronitrile
Durée de l'opération d'introduction de la base complexe : 30 min.
Température d'alkylation : 30 à 35 °C
L'huile brute obtenue titre 34,5 % en produit désiré
Rendement : 31,2 %

Spectre I.R. (film) : ≡ CH à 3 280 $cm^{-1}$
C ≡ N à 2 240 $cm^{-1}$
C ≡ C à 2 120 $cm^{-1}$
$n_D^{20}$ = 1,415 2

Exemple 35

Préparation de l'α-benzyl di-n-propylacétonitrile à partir du di-n-propylacétonitrile

a) Préparation de la base complexe amidure de sodium/éthoxy-2 éthylate de sodium

Dans un ballon de 250 ml, parfaitement sec, on introduit 8,2 g d'amidure de sodium et 66,6 g (75 ml) de tétrahydrofuranne.

A température ambiante, sous agitation et sous atmosphère d'azote, on ajoute goutte à goutte une solution de 6,3 g d'éthoxy-2 éthanol dans 22,2 g (25 ml) de tétrahydrofuranne tout en maintenant la température entre 40 et 45 °C.

On chauffe le milieu à 55/60 °C et on maintient cette température pendant 2 heures.

Après cette opération, on refroidit à la température ambiante.

b) Formation du carbanion du di-n-propylacétonitrile et alkylation avec le chlorure de benzyle

A la suspension de base complexe obtenue au paragraphe a) ci-dessus, on ajoute en 5 minutes, un mélange de 16,5 g de chlorure de benzyle et 12,5 g de di-n-propylacétonitrile.

La réaction est exothermique et l'exothermicité se maintient pendant environ 45 minutes après la fin de l'introduction. On refroidit le mélange à température ambiante et on l'y maintient pendant 1 heure.

Après quoi, on refroidit le milieu à 0/5 °C et on hydrolyse sans dépasser 20 °C par ajout de 50 g d'eau distillée. Après décantation, on élimine le solvant sous pression réduite. On reprend le concentrat dans 107 g (150 ml) d'éther éthylique et on lave la phase éthérée successivement avec 2 fractions chacune de 25 g d'une solution aqueuse à 10 % d'acide chlorhydrique et 3 fractions chacune de 25 g d'eau distillée. Après séchage sur sulfate de sodium, on élimine l'éther sous pression atmosphérique puis sous pression réduite (pression résiduelle : ≃ 50 mm Hg). De cette manière, on obtient 24,6 g d'une huile brute titrant 95,8 % en produit brut. On rectifie ensuite cette huile sous pression réduite. Rendement en α-benzyl di-n-propylacétonitrile : 75,9 %

P.E. : 108 °C sous 0,5 mm Hg
$n_D^{21}$ = 1,507 1

Spectre I.R. : C ≡ N à 2 240 $cm^{-1}$

En utilisant le même procédé que celui décrit précédemment, on a préparé les composés suivants :

Composés

α-Ethyl di-n-propylacétonitrile
L'huile brute obtenue titre 99,3 % en produit désiré
Rendement : 51,5 %
P.E. : 56 °C sous 1,7 mm Hg ou 49 °C sous 0,8 mm Hg.
$n_D^{27}$ = 1,428 8

Spectre I.R. : C ≡ N à 2 240 $cm^{-1}$
α-Allyl di-n-propylacétonitrile
L'huile brute titre 100 % en produit désiré
Rendement : 61,5 %
P.E. : 74 °C sous 1,6 mm Hg
$n_D^{22}$ = 1,441 9

Spectre I.R. : C ≡ N à 2 240 $cm^{-1}$
α-Isobutyl di-n-propylacétonitrile
L'huile brute titre 98,2 % en produit désiré
Rendement : 62,5 %
P.E. : 74-75 °C sous 1,6 mm Hg
$n_D^{21}$ = 1,437 8

Spectre I.R. : C ≡ N à 2 240 $cm^{-1}$

Exemple 36

Préparation du di-n-propylacétonitrile à partir du valéronitrile (la base complexe est ajoutée à un

mélange nitrile/halogénure-quantité totale de solvant : 6,4 volumes)

a) Préparation de la base complexe amidure de sodium/éthoxy-2 éthylate de sodium

Dans un ballon de 100 ml équipé d'un réfrigérant, d'un agitateur mécanique, d'un thermomètre de masse, d'une ampoule d'introduction isobarique munie d'une arrivée d'azote, on introduit 12,9 g (0,330 7 mole) d'amidure de sodium puis 40 ml de toluène. On agite le milieu sous atmosphère d'azote puis, par l'ampoule d'introduction, on ajoute un mélange de 9,9 g (0,110 mole) d'éthoxy-2 éthanol dans 10 ml de toluène. On porte le milieu à 60-65 °C pendant 90 minutes puis on l'agite pendant 4 à 5 heures sous atmosphère d'azote.

b) Formation du carbanion du valéronitrile et alkylation avec le bromure de n-propyle

Dans un ballon de 250 ml équipé d'un agitateur mécanique, d'un thermomètre de masse, d'un réfrigérant et d'une ampoule d'introduction isobarique munie d'une arrivée d'azote, on introduit 16,6 g ou 20,8 ml (0,2 mole) de valéronitrile, 24,6 g ou 18,2 ml (0,2 mole) de bromure de n-propyle et 90 ml de toluène. On transvase alors la base complexe dans l'ampoule isobarique et on rince le flacon de 100 ml avec 10 ml de toluène. On place alors l'ensemble de l'appareillage sous atmosphère d'azote et on agite la base complexe pour obtenir un milieu homogène. On place alors le milieu sous agitation, on préchauffe à 35 °C et on ajoute, par fractions, la suspension de base complexe. La température s'élève progressivement jusqu'à 45 °C et on la maintient à 45 ± 3 °C au moyen d'un bain-marie. L'addition nécessite environ 1 heure. On poursuit l'agitation durant 30 minutes toujours sous atmosphère d'azote puis on laisse le mélange revenir à la température ambiante. Tout en maintenant le milieu sous atmosphère d'azote, on le refroidit à + 5 à + 10 °C puis on l'hydrolyse lentement en ajoutant 40 à 50 ml d'eau. On décante la couche organique et on la lave successivement avec 60 ml d'acide chlorhydrique à 20 % (en volume) puis avec 4 fractions chacune de 60 ml d'eau. On sèche la phase organique sur sulfate de sodium et on filtre.

De cette manière, on obtient le di-n-propylacétonitrile avec un rendement de 83,82 % ainsi que le valéronitrile et le tri-n-propylacétonitrile avec des rendements respectifs de 5,95 % et 2,47 %.

En utilisant le même procédé que celui décrit précédemment, mais en tenant compte des variations indiquées ci-dessus, on a préparé le di-n-propylacétonitrile avec les résultats suivants :

| Solvant | Conditions opératoires: T° de réaction (°C) | Durée de l'addition de la base complexe (min) | Rendement en %: I (*) | II (**) | III (***) |
|---|---|---|---|---|---|
| Benzène (6,4 vol.) | 45 ± 3 | 30 | 8,22 | 82,45 | 2,4 |
| Cyclohexane (6,4 vol.) | 45 ± 3 | 45 | 1,66 | 85,14 | 2,68 |
| Ether diisopropylique (6,4 vol.) | 40-45 | 60 | 4,6 | 87,1 | 3 |

(*) I = valéronitrile
(**) II = di-n-propylacétonitrile
(***) III = tri-n-propylacétonitrile

## Exemple 37

Préparation du di-n-propylacétonitrile à partir du valéronitrile (un mélange de nitrile/halogénure est ajouté à la base complexe)

A. La base complexe est en suspension dans la quantité totale de solvant c'est-à-dire 6,4 volumes

a) Préparation de la base complexe amidure de sodium/éthoxy-2 éthylate de sodium

Dans un flacon de 250 ml équipé d'un agitateur, d'un réfrigérant, d'un thermomètre de masse et

d'une ampoule d'introduction isobarique munie d'une arrivée d'azote, on introduit 12,9 g (0,3307 mole) d'amidure de sodium et 100 ml d'éther diisopropylique. On mélange le milieu sous atmosphère d'azote puis on ajoute 9,9 g (0,110 mole) d'éthoxy-2 éthanol dans 60 ml d'éther diisopropylique. On chauffe le milieu à 60 °C pendant 90 minutes et on maintient l'agitation à température ambiante pendant 4 à 5 heures.

b) Formation du carbanion du valéronitrile et alkylation avec le bromure de n-propyle

A la suspension de base complexe ainsi obtenue, on ajoute, goutte à goutte, un mélange de 16,6 g ou 20,8 ml (0,2 mole) de valéronitrile et 24,6 g ou 18,2 ml (0,2 mole) de bromure de n-propyle tout en maintenant une température de réaction de 20 à 25 °C au moyen d'un bain-marie (environ 10 °C). L'addition nécessite 25 minutes. On agite à nouveau le milieu à 25 °C pendant 30 minutes sous atmosphère d'azote puis on refroidit à + 5 °C. On hydrolyse lentement avec 50 ml d'eau, on décante le mélange et on lave successivement avec 60 ml d'acide chlorhydrique à 20 % (volume) puis avec 4 fractions chacune de 60 ml d'eau distillée. On sèche la couche organique et on concentre sous pression atmosphérique.

De cette manière, on prépare 23,9 g de di-n-propylacétonitrile brut. Ainsi, le di-n-propylacétonitrile a été obtenu avec un rendement de 78,2 % ainsi que le valéronitrile et le tri-n-propylacétonitrile avec des rendements respectifs de 8,8 % et 5,7 %.

En utilisant le même procédé que celui décrit précédemment, mais en tenant compte des variations indiquées ci-dessus, on a préparé le di-n-propylacétonitrile avec les résultats suivants :

| Solvant | Conditions opératoires | | Rendements en % | | |
|---|---|---|---|---|---|
| | T° de réaction (°C) | Durée de l'addition de la base complexe (min) | I (*) | II (**) | III (***) |
| Toluène (6,4 vol.) | 30-35 | 20 | 6,6 | 79,65 | 3,75 |
| Benzène (5,6 vol.) | 30-35 | 20 | 13 | 71,8 | 4,8 |
| Cyclohexane (6,4 vol.) | 28-30 | 10 | 13,3 | 76 | 2,8 |
| Benzène (4,8 vol.) | 30-35 | 32 | 7,6 | 78,8 | 6 |

(*) I = valéronitrile
(**) II = di-n-propylacétonitrile
(***) III = tri-n-propylacétonitrile

B. Le nitrile est dans 3 volumes de solvant et la base complexe dans 3,6 volumes de solvant

a) Préparation de la base complexe amidure de sodium/éthoxy-2 éthylate de sodium

Dans un ballon de 250 ml équipé d'un agitateur, d'un réfrigérant, d'un thermomètre de masse et d'une ampoule d'introduction isobarique munie d'une arrivée d'azote, on introduit 12,9 g (0,3307 mole) d'amidure de sodium puis 60 ml d'éther diisopropylique. On agite le mélange sous atmosphère d'azote et on ajoute, goutte à goutte, 9,9 g (0,110 mole) d'éthoxy-2 éthanol dilués dans 15 ml d'éther diisopropylique. On porte le mélange à 60 °C pendant 90 minutes puis on mélange pendant 5 heures à température ambiante. Après cette opération, on ajoute 15 ml d'éther diisopropylique.

b) Formation du carbanion du valéronitrile et alkylation avec le bromure de n-propyle

A la base complexe ainsi obtenue, on ajoute à 25 °C un mélange de 16,6 g (0,2 mole) de valéronitrile et 24,6 g (0,2 mole) de bromure de n-propyle dans 75 ml d'éther diisopropylique. L'addition nécessite 25 minutes à 30 ± 2 °C, cette température étant maintenue au moyen d'un bain-marie. On agite alors le milieu à 30 °C pendant 30 minutes, refroidit à 5 °C et hydrolyse lentement avec 50 ml d'eau. Après décantation, on lave le milieu avec de l'acide chlorhydrique à 20 % puis avec 4 fractions chacune de 60 ml

d'eau. On sèche la phase organique et on la concentre à la pression atmosphérique. De cette manière, on prépare 29,9 g de di-n-propylacétonitrile brut.

Ainsi, le di-n-propylacétonitrile a été obtenu avec un rendement de 74,75 % ainsi que le valéronitrile et le tri-n-propylacétonitrile avec des rendements respectifs de 9 % et 6,44 %.

En utilisant le même procédé que celui décrit précédemment, mais en tenant compte des variations indiquées ci-dessus, on a préparé le di-n-propylacétonitrile avec les résultats suivants :

| Solvant | Conditions opératoires: T° de réaction (°C) | Conditions opératoires: Durée de l'addition de la base complexe (min) | Rendements en %: I (*) | Rendements en %: II (**) | Rendements en %: III (***) |
|---|---|---|---|---|---|
| Toluène (6,6 vol.) | 30-35 | 30 | 12,2 | 70,6 | 1,82 |

## Exemple 38

Préparation du di-n-propylacétonitrile à partir de l'acétonitrile

A. Addition de la base complexe à un mélange d'acétonitrile/bromure de n-propyle-quantité totale de solvant : 10,8 volumes.

a) Préparation de la base complexe amidure de sodium/éthoxy-2 éthylate de sodium.

Dans un ballon de 100 ml équipé d'un réfrigérant, d'un agitateur mécanique, d'un thermomètre de masse et d'une ampoule d'introduction isobarique munie d'une arrivée d'azote, on introduit 12,9 g (0,330 7 mole) d'amidure de sodium et 40 ml de toluène. On agite le mélange sous atmosphère d'azote et on ajoute une solution de 9,9 g (0,110 mole) d'éthoxy-2 éthanol dans 20 ml de toluène. On porte le milieu à 60-65 °C pendant 90 minutes et on le maintient sous agitation et atmosphère d'azote pendant 4 à 5 heures.

b) Formation du carbanion de l'acétonitrile et alkylation avec le bromure de n-propyle

Dans un ballon de 250 ml équipé d'un réfrigérant, d'un agitateur, d'un thermomètre, d'une ampoule isobarique d'introduction munie d'une arrivée d'azote, on place 4,1 g (0,1 mole) d'acétonitrile, 24,6 g (0,2 mole) de bromure de n-propyle et 85 ml de toluène. On transvase alors la base complexe dans l'ampoule d'introduction et on rince le ballon de 100 ml avec 15 ml de toluène. On place alors la totalité de l'appareillage sous atmosphère d'azote et on agite la base complexe ainsi obtenue de façon à obtenir un milieu homogène. On agite alors le milieu réactionnel et on le préchauffe à 35 °C. Après cette opération, on ajoute, par fractions, la suspension de base complexe tout en maintenant la température entre 40 et 45 °C au moyen d'un bain-marie glacé. L'opération d'addition nécessite 22 minutes après quoi on maintient la température à 45 °C. On laisse le milieu réactionnel revenir à la température ambiante et on maintient l'agitation pendant 30 minutes. Après refroidissement à + 5 °C sous atmosphère d'azote, on hydrolyse lentement le milieu avec 40 à 50 ml d'eau. On décante le mélange et on lave la phase organique avec 60 ml d'acide chlorhydrique à 20 % (en volume) puis avec 4 fractions chacune de 60 ml d'eau. On sèche la couche organique sur sulfate de sodium et on filtre.

De cette manière, on obtient le di-n-propylacétonitrile avec un rendement de 65,9 % ainsi que le valéronitrile et le tri-n-propylacétonitrile avec des rendements respectifs de 25,9 % et 3 %.

En utilisant le même procédé que celui décrit précédemment, mais en tenant compte des variations indiquées ci-dessus, on a préparé le di-n-propylacétonitrile avec les résultats suivants :

(Voir tableau, page 20)

| Solvant | Conditions opératoires | | Rendements en % | | |
| --- | --- | --- | --- | --- | --- |
| | T° de réaction (°C) | Durée de l'addition de la base complexe (min.) | I (*) | II (**) | III (***) |
| Ether diisopropylique (10,8 vol.) | 35 | 60 | 21,5 | 56,8 | 2,1 |
| Cyclohexane (12,8 vol.) | 40 ± 2 | 55 | - | 50,5 | 10 |

(*) I = valéronitrile
(**) II = di-n-propylacétonitrile
(***) III = tri-n-propylacétonitrile

B. Addition d'un mélange acétonitrile/bromure de n-propyle à la base complexe-quantité totale de solvant : 18,6 volumes

a) Préparation de la base complexe amidure de sodium/éthoxy-2 éthylate de sodium

Dans un ballon de 250 ml équipé d'un réfrigérant, d'un agitateur mécanique, d'un thermomètre de masse et d'une ampoule d'introduction munie d'une arrivée d'azote, on introduit 12,9 g d'amidure de sodium et 100 ml d'éther diisopropylique. On agite le mélange sous atmosphère d'azote et on ajoute une solution de 9,9 g (0,110 mole) d'éthoxy-2 éthanol. On porte alors le milieu à 60 °C pendant 90 minutes et on le maintient sous agitation et atmosphère d'azote pendant 4 à 5 heures.

b) Formation du carbanion de l'acétonitrile et alkylation avec le bromure de n-propyle

On refroidit à 15 °C la base complexe obtenue précédemment et on y ajoute rapidement par l'ampoule d'introduction, une solution de 4,1 g (0,1 mole) d'acétonitrile et 24,6 g (0,2 mole) de bromure de n-propyle. Cette addition nécessite 10 minutes à 15 à 20 °C, cette température étant maintenue au moyen d'un bain-marie. On rince l'ampoule avec 10 ml d'éther diisopropylique et on agite le milieu réactionnel pendant 20 minutes à 25 °C. Après refroidissement à + 5 °C, on hydrolyse lentement avec 50 ml d'eau et on décante. On lave la couche organique successivement avec 60 ml d'acide chlorhydrique à 20 % (en volume) puis avec 4 fractions chacune de 60 ml d'eau distillée. Après séchage sur sulfate de sodium, on concentre la phase organique sous pression atmosphérique.

De cette manière, on obtient 11,75 g de di-n-propylacétonitrile brut. Ainsi, le di-n-propylacétonitrile a été obtenu avec un rendement de 65,2 % ainsi que le valéronitrile et le tri-n-propylacétonitrile avec des rendements respectifs de 16 % et 7,3 %.

En utilisant le même procédé que celui décrit précédemment, mais en tenant compte des variations indiquées ci-dessus, on a préparé le di-n-propylacétonitrile avec les résultats suivants :

| Solvant | Conditions opératoires | | Rendements en % | | |
| --- | --- | --- | --- | --- | --- |
| | T° de réaction (°C) | Durée d'addition de la base complexe (min.) | I (*) | II (**) | III (***) |
| Toluène (8 vol.) | 10-30 | 16 | 22 | 70,7 | 4,9 |

(*) I = valéronitrile
(**) II = di-n-propylacétonitrile
(***) III = tri-n-propylacétonitrile

**Revendications**

1. Procédé de préparation de composés de formule générale

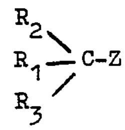

I

dans laquelle Z représente un radical nitrile, un radical acide carboxylique simple ou estérifié par un radical alkyle, linéaire ou ramifié, ayant de 1 à 5 atomes de carbone ou un radical amide tertiaire de formule générale

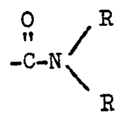

dans laquelle

R représente un radical alkyle linéaire ayant de 1 à 3 atomes de carbone

$R_1$ représente hydrogène ou un radical alkyle, linéaire ou ramifié, ayant de 1 à 6 atomes de carbone

$R_2$ représente un radical alkyle, linéaire ou ramifié, ayant de 1 à 12 atomes de carbone, un radical cycloalkyle ou un radical aralkyle dans lesquels le reste alkyle compte de 1 à 4 atomes de carbone et

$R_3$ représente hydrogène ou l'un des radicaux définis pour $R_2$ ci-dessus, caractérisé en ce que :

— dans une première étape, on fait réagir une chaîne carbonée de formule générale

$$R_1{-}CH_2{-}Z \qquad II$$

dans laquelle $R_1$ a la même signification que précédemment, avec une base complexe constituée par un mélange d'un amidure de métal alcalin et d'un alcoolate de métal alcalin en suspension dans un solvant organique anhydre, de manière à donner naissance transitoirement à un carbanion,

— puis dans une seconde étape, on fait réagir ce carbanion dans un solvant organique anhydre avec un halogénure d'alkyle de formule générale

$$R_2X$$

dans laquelle X représente un atome d'halogène et $R_2$ a la même signification que précédemment pour obtenir les composés de formule I dans laquelle $R_3$ représente hydrogène ou un radical identique à $R_2$ et on répète les deux étapes, avec pour la seconde, la réaction avec un halogénure de formule générale

$$R_3X$$

dans laquelle $R_3$ a la même signification que précédemment à l'exception d'hydrogène et X a la même signification que précédemment, pour obtenir les composés de formule I dans laquelle $R_3$ représente un radical identique à ou différent de $R_2$.

2. Procédé selon la Revendication 1 caractérisé en ce que, en vue de fixer deux substituants identiques sur la chaîne carbonée de formule II, on opère en un seul couple d'étapes, les proportions des réactifs utilisés étant modifiées de manière, d'une part à provoquer successivement *in situ* la formation de deux carbanions et, d'autre part, à assurer un excès notable de l'halogénure d'alkyle.

3. Procédé selon l'une quelconque des Revendications 1 ou 2 caractérisé en ce que l'on introduit la suspension de base complexe dans une solution de composé de formule II et d'halogénure d'alkyle à une température comprise entre 0 et 72 °C, de préférence entre 10 et 20 °C.

4. Procédé selon l'une quelconque des Revendications 1 ou 2 caractérisé en ce que l'on introduit la solution de composé de formule II et d'halogénure d'alkyle dans la suspension de base complexe à une température comprise entre − 10 et + 20 °C.

5. Procédé selon l'une quelconque des Revendications 1 à 4 caractérisé en ce que le solvant organique anhydre est le tétrahydrofuranne ou l'éther diisopropylique.

6. Procédé selon les Revendications 1 et 2 caractérisé en ce que la chaîne carbonée de formule II est l'acétonitrile et le composé de formule I obtenu est le di-n-propylacétonitrile.

7. Procédé selon la Revendication 1 caractérisé en ce que la chaîne carbonée de formule II est le valéronitrile et le composé de formule I obtenu est le di-n-propylacétonitrile.

8. Procédé selon les Revendications 1 et 2 caractérisé en ce que la chaîne carbonée de formule II est l'acide acétique et le composé de formule I obtenu est l'acide di-n-propylacétique.

9. Procédé selon la Revendication 1 caractérisé en ce que la chaîne carbonée de formule II est l'acide valérique et le composé de formule I obtenu est l'acide di-n-propylacétique.

10. Procédé selon la Revendication 1 caractérisé en ce que le tri-n-propylacétonitrile est obtenu au départ de di-n-propylacétonitrile.

11. Procédé selon l'une quelconque des Revendications précédentes caractérisé en ce que l'halogénure d'alkyle est un chlorure d'alkyle ou un bromure d'alkyle.

12. Procédé selon l'une quelconque des Revendications précédentes caractérisé en ce que le mélange d'amidure de métal alcalin et d'alcoolate de métal alcalin correspond à la formule symbolique

$$MNR_4/R_5OM'$$

dans laquelle $R_4$ représente $H_2$, $(C_2H_5)_2$ ou $(iso\text{-}C_3H_7)_2$, $R_5$ représente un radical alkyle linéaire ou ramifié, ayant de 1 à 7 atomes de carbone ou encore un radical $C_2H_5—O—CH_2—CH_2—$ ou $CH_3—O—CH_2—CH_2—$ et M et M', qui sont identiques ou différents, représentent chacun un métal alcalin tel que le lithium, le sodium ou le potassium.

13. Procédé selon la Revendication 12, caractérisé en ce que le mélange d'amidure de métal alcalin et d'alcoolate de métal alcalin est choisi entre amidure de sodium/t-butylate de sodium ; amidure de sodium/isopropylate de sodium ; amidure de sodium/n-propylate de sodium ; amidure de sodium/éthoxy-2 éthylate de sodium ; amidure de sodium/t-amylate de sodium ; amidure de sodium/t-butylate de potassium ; amidure de lithium/t-butylate de potassium ; diéthylamidure de lithium/t-amylate de lithium et amidure de lithium/t-amylate de lithium.

14. Procédé selon la Revendication 12 ou 13 caractérisé en ce que l'amidure de métal alcalin et l'alcoolate de métal alcalin sont dans le rapport 2 : 1 ou le rapport 3 : 1 ou le rapport 5 : 1.

**Claims**

1. Process for preparing compounds of the general formula

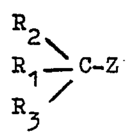

I

in which Z represents a nitrile radical, a carboxylic acid radical free or esterified by a straight- or branched-chain alkyl radical having from 1 to 5 carbon atoms or a tertiary amide radical of general formula

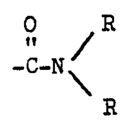

in which

R represents a straight-chain alkyl radical having from 1 to 3 carbon atoms,

$R_1$ represents hydrogen or a straight- or branched-chain alkyl radical having from 1 to 6 carbon atoms,

$R_2$ represents a straight- or branched-chain alkyl radical having from 1 to 12 carbon atoms, a cycloalkyl radical or an aralkyl radical in which the alkyl moiety has from 1 to 4 carbon atoms,

$R_3$ represents hydrogen or one of the radicals as defined for $R_2$ above, whereby :

— in a first step, a carbon chain of the general formula

$$R_1—CH_2—Z \qquad II$$

in which $R_1$ has the same meaning as given above is reacted with a complex base comprising a mixture of alkali metal amide and alkali metal alcoholate suspended in an anhydrous organic solvent, to provide temporarily a carbanion,

— then, in a second step, the carbanion is reacted in an anhydrous organic solvent with an alkyl halide of the general formula

$$R_2X$$

in which X represents a halogen atom and $R_2$ has the same meaning as given above to obtain the compounds of formula I in which $R_3$ represents hydrogen or a radical identical to $R_2$ and the two steps are repeated, the second one involving the reaction with a halide of general formula

$$R_3X$$

in which $R_3$ has the same meaning as given above with the exception of hydrogen and X has the same meaning as given above, to obtain the compounds of formula I in which $R_3$ represents a radical identical to or different from $R_2$.

2. Process according to Claim 1 whereby one single pair of successive steps is carried out to add two identical substituents to the carbon chain of formula II, the proportions of the reagents being such that two carbanions are successively formed *in situ* and there is provided a considerable excess of alkyl halide.

3. Process according to Claim 1 or 2, whereby the suspension of complex base is introduced into a solution of a compound of formula II and of alkyl halide at a temperature of 0 to 72 °C and preferably 10 to 20 °C.

4. Process according to Claim 1 or 2 whereby the solution of a compound of formula II and of alkyl halide is introduced into the suspension of complex base at a temperature of − 10 to + 20 °C.

5. Process according to any of Claims 1 to 4 whereby the anhydrous organic solvent is tetrahydrofuran or diisopropyl ether.

6. Process according to Claims 1 and 2 whereby the carbon chain of formula II is acetonitrile and the compound of formula I so obtained is di-n-propylacetonitrile.

7. Process according to Claim 1 whereby the carbon chain of formula II is valeronitrile and the compound of formula I so obtained is di-n-propylacetonitrile.

8. Process according to Claims 1 and 2 whereby the carbon chain of formula II is acetic acid and the compound of formula I so obtained is di-n-propylacetic acid.

9. Process according to Claim 1 whereby the carbon chain of formula II is valeric acid and the compound of formula I so obtained is di-n-propylacetic acid.

10. Process according to Claims 1 and 2 whereby tri-n-propylacetonitrile is obtained from di-n-propylacetonitrile.

11. Process according to any of the preceding Claims whereby the alkyl halide is an alkyl chloride or alkyl bromide.

12. Process according to any of the preceding Claims whereby the mixture of alkali metal amide and alkali metal alcoholate corresponds to the symbolic formula

$$MNR_4/R_5OM'$$

in which $R_4$ represents $H_2$, $(C_2H_5)_2$ or $(iso\text{-}C_3H_7)_2$, $R_5$ represents a straight- or branched-chain alkyl radical having from 1 to 7 carbon atoms or a radical $C_2H_5—O—CH_2—CH_2—$ or $CH_3—O—CH_2—CH_2—$ and M and M', which are the same or different, each represent an alkali metal such as lithium, sodium or potassium.

13. Process according to Claim 12 whereby the mixture of alkali metal amide and alkali metal alcoholate is selected from sodium amide/sodium t-butylate ; sodium amide/sodium isopropylate ; sodium amide/sodium n-propylate ; sodium amide/sodium 2-ethoxy-ethylate ; sodium amide/sodium t-amylate ; sodium amide/potassium t-butylate ; lithium amide/potassium t-butylate ; lithium diethylamide/lithium t-amylate and lithium amide/lithium t-amylate.

14. Process according to Claim 12 or 13 whereby the alkali metal amide and the alkali metal alcoholate are in a 2 : 1 ratio or 3 : 1 ratio or 5 : 1 ratio.

**Ansprüche**

1. Verfahren zur Herstellung von Verbindungen der allgemeinen Formel

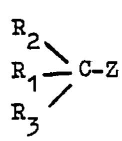

I

in welcher Z für einen Nitrilrest, einen freien oder durch einen gerade- oder verzweigtkettigen Alkylrest mit 1 bis 5 Kohlenstoffatomen veresterten Carbonsäurerest oder einen tertiären Amidrest der allgemienen Formel

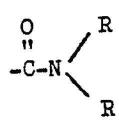

steht, in welcher

R für einen geradekettigen Alkylrest mit 1 bis 3 Kohlenstoffatomen steht,

$R_1$ für Wasserstoff oder einen gerade- oder verzweigtkettigen Alkylrest mit 1 bis 6 Kohlenstoffatomen steht,

$R_2$ für einen gerade- oder verzweigtkettigen Alkylrest mit 1 bis 12 Kohlenstoffatomen, einen Cycloalkylrest oder einen Aralkylrest, in dem der Alkylteil 1 bis 4 Kohlenstoffatomen enthält, steht,

$R_3$ für Wasserstoff oder einen der oben für $R_2$ angegebenen Reste steht, dadurch gekennzeichnet, daß

— in einer ersten Stufe eine Kohlenstoffkette der allgemeinen Formel

$$R_1—CH_2—Z \qquad II$$

in der $R_1$ die oben angegebene Bedeutung hat, mit einer Komplexbase, die aus einer Mischung eines Alkalimetallamids und Alkalimetallalkoholates, suspendiert in einem wasserfreien organischen Lösungsmittel, besteht, zur zeitweiligen Schaffung eines Carbanions umgesetzt wird, dann in einer zweiten Stufe das Carbanion in einem wasserfreien organischen Lösungsmittel mit einem Alkylhalogenid der allgemeinen Formel

$$R_2X$$

in welcher X für ein Halogenatom steht und $R_2$ die oben angebene Bedeutung hat, zur Bildung der Verbindung der Formel I, in welcher $R_3$ für Wasserstoff oder einem mit $R_2$ identischen Rest steht, umgesetzt wird und die beiden Stufen wiederholt werden, wobei die zweite Reaktion mit einem Halogenid der allgemeinen Formel

$$R_3X$$

erfolgt, in welcher $R_3$ die oben angegebene Bedeutung mit der Ausnahme von Wasserstoff hat und X die oben angegebene Bedeutung hat, um die Verbindungen der Formel I zu erhalten, in welchen $R_3$ für einen mit $R_2$ identischen oder von diesem verschiedenen Rest steht.

2. Verfahren gemäß Anspruch 1, dadurch gekennzeichnet, daß ein einziges Paar aufeinanderfolgender Stufen durchgeführt wird, um zwei identische Substituenten an die Kohlenstoffkette der Formel II zu addieren, wobei die Verhältnisse der Reaktionsteilnehmer so beschaffen sind, daß zwei Carbanione nacheinander in situ gebildet werden und ein erheblicher Alkylhalogenidüberschuß vorgesehen wird.

3. Verfahren nach Anspruch 1 oder 2, dadurch gekennzeichnet, daß die Suspension der Komplexbase in eine Lösung aus einer Verbindung der Formel II und einem Alkylhalogenid bei einer Temperatur von 0 bis 72 °C und vorzugsweise 10 bis 20 °C, eingeführt wird.

4. Verfahren nach Anspruch 1 oder 2, dadurch gekennzeichnet, daß die Lösung einer Verbindung der Formel II und eines Alkylhalogenids in die Suspension der Komplexbase bei einer Temperatur von $-10$ bis $+20$ °C eingeführt wird.

5. Verfahren nach Anspruch 1 bis 4, dadurch gekennzeichnet, daß das wasserfreie organische Lösungsmittel Tetrahydrofuran oder Diisopropylether ist.

6. Verfahren nach Anspruch 1 und 2, dadurch gekennzeichnet, daβ die Kohlenstoffkette der Formel II Acetonitril und die so erhaltene Verbindung der Formel I Di-n-propylacetonitril ist.

7. Verfahren nach Anspruch 1, dadurch gekennzeichnet, daβ die Kohlenstoffkette der Formel II Valeronitril und die so erhaltene Verbindung der Formel I Di-n-propylacetonitril ist.

8. Verfahren nach Anspruch 1 und 2, dadurch gekennzeichnet, daβ die Kohlenstoffkette der Formel II Essigsäure und die so erhaltene Verbindung der Formel I Di-n-propylessigsäure ist.

9. Verfahren nach Anspruch 1, dadurch gekennzeichnet, daβ die Kohlenstoffkette der Formel II Valeriansäure und die so erhaltene Verbindung der Formel I Di-n-propylessigsäure ist.

10. Verfahren nach Anspruch 1 und 2, dadurch gekennzeichnet, daß Tri-n-propylacetonitril aus Di-n-propylacetonitril erhalten wird.

11. Verfahren gemäβ einem der vorhergehenden Ansprüche, dadurch gekennzeichnet, daβ das Alkylhalogenid ein Alkylchlorid oder Alkylbromid ist.

12. Verfahren nach einem der vorhergehenden Ansprüche, dadurch gekennzeichnet, daβ die Mischung aus Alkalimetallamid und Alkalimetallalkoholat der symbolischen Formel

$$MNR_4/R_5OM'$$

entspricht, in welcher $R_4$ für $H_2$, $(C_2H_5)_2$ oder $(iso\text{-}C_3H_7)_2$, steht, $R_5$ für einen gerade- oder verzweigtkettigen Alkylrest mit 1 bis 7 Kohlenstoffatomen oder einen Rest $C_2H_5—O—CH_2—CH_2—$ oder $CH_3—O—CH_2CH_2—$ steht und M und M', die gleich oder verschieden sein können, jeweils für ein Alkalimetall, wie Lithium, Natrium oder Kalium, stehen.

13. Verfahren nach Anspruch 12, dadurch gekennzeichnet, daβ die Mischung aus Alkalimetallamid und Alkalimetallalkoholat ausgewählt ist aus Natriumamid/Natrium-tert.- butylat ; Natriumamid/Natriumisopropylat, Natriumamid/Natrium-n-propylat ; Natriumamid/Natrium-2-ethoxyethylat ; Natriumamid/Natrium-tert.-amylat ; Natriumamid/Kalium-tert.-butylat ; Lithiumamid/Kalium-tert.-butylat, Lithiumdiethylamid/Lithium-tert.-amylat und Lithiumamid/Lithium-tert.-amylat.

14. Verfahren nach Anspruch 12 oder 13, dadurch gekennzeichnet, daß das Alkalimetallamid und das Alkalimetallalkoholat in einem 2 : 1 Verhältnis oder 3 : 1 Verhältnis oder 5 : 1 Verhältnis vorliegen.